# EUROPEAN PATENT SPECIFICATION

(11) **EP 4 615 556 B1**
(45) Date of publication and mention of the grant of the patent: **04.11.2026**
(21) Application number: 23783152.4
(22) Date of filing: 07.09.2023
(51) Int. Cl.: A61M 39/26

(54) **FLUID CONNECTOR ASSEMBLY WITH NEUTRAL FLUID DISPLACEMENT THAT LIMITS CONNECTOR DAMAGE**
FLUIDVERBINDERANORDNUNG MIT NEUTRALER FLUIDVERDRÄNGUNG ZUR BEGRENZUNG VON VERBINDERSCHÄDEN
ENSEMBLE RACCORD DE FLUIDE À DÉPLACEMENT DE FLUIDE NEUTRE QUI LIMITE LES DOMMAGES DU RACCORD

(30) Priority: 08.11.2022 US 202217982718
(43) Date of publication of application: 17.09.2025
(73) Proprietor: CareFusion 303, Inc., San Diego, CA 92130 (US)
(72) Inventor: JADHAV, Amarsinh Deeliprao, Bengaluru 560 045 Karnataka (IN); RAJPAL, Sumit, Bengaluru 560 045 Karnataka (IN); SUMAN, Kaushik, Bengaluru 560 045 Karnataka (IN); AUSTIN, Abin, Bengaluru 560 045 Karnataka (IN); MENON, Kanjimpuredathil Muralikrishna, Bengaluru 560 045 Karnataka (IN)
(74) Representative: Zenz Patentanwälte Partnerschaft mbB
(86) International application number: PCT/US2023/032210
(87) International publication number: WO 2024/102192

(56) References cited:
- US-A1- 2016 129 235
- US-B1- 6 299 131
- US-B2- 7 044 441

## Description

### TECHNICAL FIELD

The present disclosure relates generally to medical fluid connectors and, more particularly, to neutral displacement needle-free connectors that maintain separation between a central post and a luer to reduce the likelihood of the luer breaking the central post.

### BACKGROUND

Needle-free connectors, including neutral displacement needle-free connectors, offer a solution for providing medical fluid to patients. In an exemplary embodiment, a needle-free connector assembly promotes fluid transmission between a medical fluid supply and a catheter line. The medical fluid supply and the catheter line are secured to respective luers that are secured to a connector body. To transmit fluid, the luer connected to the medical supply is inserted into the connector body and overlaps a central post of the luer connected to the catheter line.

US 6 299 131 B1 discloses a needleless IV injection port assembly including a spiked body provided with a hollow spike, a female luer component, a flexible and resilient spike boot having a tip and spring portion, a centering member, and a resilient swabbable septum. The spike boot extends over the spike, the centering member is provided over a portion of the spike and spike boot, and the septum is provided between the centering member and an end of the female luer component. The spiked body and the female luer component are secured together to complete the assembly. The length of the female luer component and/or the length of the spike is chosen so that when a separate male luer is positioned over a protuberance on the septum and pushed into the female luer component, the male luer places pressure upon the septum which thereby urges the septum toward the spike. The pressure on the septum causes the spring portion of the boot to be compressed and the tip portion of the boot to be pierced by the tip of the spike. As the septum is further compressed, the septum extends over the spike, with a pre-slit hole in the septum facilitating the extension thereover, such that a hollow of the spike is in fluid communication with the male luer connector. The injection port causes minimal reflux, and the spike boot and septum provide a swabbable antibacterial double barrier over the spike.

US 7 044 441 B2 discloses a male Luer connector that attaches to a standard female Luer connector to open a medical fluid flow channel between the two connectors. The male connector has two internal valves and a vacuum-generating structure. The two valves and the vacuum-generating structure are configured to draw back into the male connector any fluid residing on the interface between the male and female connectors during their disengagement from one another and to then seal the distal tip of the male connector. One valve is located at the proximal end of the male connector while another is located at the distal, interface end of the male connector. Both valves are opened by an actuator plug that is moved to open and close the valves by contact with the front surface of the female connector. A resilient member biases the male valves to the closed position and includes a variable internal volume that creates the partial vacuum upon disengagement of the male and female connectors. The male valves are timed to a particular sequence with the closing of an internal female connector valve so that the partial vacuum generated by the male connector will have the most fluid drawback effect.

US 2016/129235 A1 discloses n injection port assembly including a body having first and second mating structures configured to mate with a first connector for a first fluid pathway and a second connector on a device, respectively. A resilient barrier substantially contained within the body and compressible from a first position in which fluid flow between the first and second connectors is blocked to a more compressed second position in which fluid flow between the first and second connectors is permitted. A hollow cannula can be coupled with the first mating structure and disposed within the resilient barrier, the hollow cannula having a distal end configured to extend through the resilient barrier when the resilient barrier is in the second position, the distal end having lateral slots. The resilient barrier can have first and second annular sealing rings positioned above and below the lateral slots respectively.

The overlapping between the luer and the central post can lead to issues. For example, movement of the luer relative to the central post, or vice versa, can cause contact between the luer and the central post, leading to increased risk of cracking or breaking the central post. When this occurs, the damaged area leads to fluid leakage, resulting in fluid loss and contamination. Additionally, luers used with neutral displacement connectors are often non-compliant luers, making them susceptible to fluid leakage.

### SUMMARY

The invention is defined in the independent claims. Preferable embodiments are further laid out in the dependent claims. In accordance with at least some embodiments disclosed herein is the realization that components used with neutral fluid displacement, such as a luer and a post, can break when overlapping and coming into contact with each other, which can result in medical fluid leakage and reduced medical delivery to a patient. When a broken component is located in a connector body, it may not be readily present to a medical professional that the component is broken, leading to delayed response times.

Aspects of the present disclosure provide a needle-free fluid connector assembly that provides a neutral fluid displacement connection while minimizing the risk of breaking one or more components of the needle-free fluid connector assembly. The neutral fluid displacement limits or prevents fluid from entering a catheter lumen during connection or disconnection of a medical fluid. Based on the positioning of respective posts of the connectors of the fluid connector assembly, the posts do not overlap each other.

Accordingly, aspects of the present disclosure provide a fluid connector assembly comprising a housing, comprising a first end, a second end, and an internal volume fluidly connected to the first end and the second end, and a compressible member disposed in the internal volume, the compressible member comprising a cavity; a first connector secured with the housing at the first end, the first connector comprising: a post located in the cavity, and an opening formed in the post; and a second connector, when secured with the housing at second end, displaces the compressible member and forms a seal that causes a fluid entering the second connector to pass through the opening.

Some instances of the present disclosure provide a fluid connector assembly comprising a fluid connector assembly, comprising a housing comprising an internal volume; a compressible member disposed in the internal volume, the compressible member comprising: an inner wall, and a cavity defined by the inner wall; a first connector secured with the housing, the first connector comprising a post located in the cavity, and an opening formed in the post, the opening covered by the inner wall; and a second connector, wherein: a first position of the second compressible member comprises the inner wall covering the opening, and a second position of the second compressible member comprises the inner wall uncovering the opening based upon the second connector.

Some instances of the present disclosure provide a method for regulating an intravenous fluid, by a fluid connector assembly, comprising providing a housing, the housing comprising an in internal volume; receiving, at the housing, a compressible member in the internal volume; receiving, at a first end of the housing, a first connector, the first connector comprising a post and an opening formed in the post; receiving, at a second end of the housing, a second connector; displacing, based on receiving the second connector, the compressible member; and forming, based on the displacing of the compressible member, a seal that causes the intravenous fluid that enters the second connector to pass through the opening.

Accordingly, the present application addresses several operational challenges encountered in prior neutral displacement connector assemblies that are susceptible to breaking.

Additional features and advantages of the subject technology will be set forth in the description below, and in part will be apparent from the description, or may be learned by practice of the subject technology. The advantages of the subject technology will be realized and attained by the structure particularly pointed out in the written description and embodiments hereof as well as the appended drawings.

It is to be understood that both the foregoing general description and the following detailed description are exemplary and explanatory and are intended to provide further explanation of the subject technology.

### BRIEF DESCRIPTION OF THE DRAWINGS

Various features of illustrative embodiments of the inventions are described below with reference to the drawings. The illustrated embodiments are intended to illustrate, but not to limit, the inventions. The drawings contain the following figures:
FIG. 1 illustrates an IV set coupled to a patient, in accordance with aspects of the present disclosure.
FIG. 2 illustrates a perspective view of a fluid connector assembly for in use with an IV set, in accordance with aspects of the present disclosure.
FIG. 3 illustrates an exploded view of the fluid connector assembly, showing additional features, in accordance with aspects of the present disclosure.
FIG. 4 illustrates a partial cross-sectional view of the fluid connector assembly, showing the connector and the compressible member positioned in the housing, in accordance with aspects of the present disclosure.
FIG. 5 illustrates a partial cross-sectional view of the fluid connector assembly, showing the connector inserted into the housing, in accordance with aspects of the present disclosure.
FIG. 6 illustrates a partial cross-sectional view of the fluid connector assembly, showing the connector removed from the housing, in accordance with aspects of the present disclosure.
FIG. 7 illustrates a partial cross-sectional view of an alternate embodiment of a fluid connector assembly, showing a biasing component integrated with the fluid connector assembly, in accordance with aspects of the present disclosure.
FIG. 8 illustrates a flowchart showing a method for regulating an intravenous fluid, in accordance with aspects of the present disclosure.

### DETAILED DESCRIPTION

In the following detailed description, numerous specific details are set forth to provide a full understanding of the subject technology. It should be understood that the subject technology may be practiced without some of these specific details. In other instances, well-known structures and techniques have not been shown in detail so as not to obscure the subject technology.

Further, while the present description sets forth specific details of various embodiments, it will be appreciated that the description is illustrative only and should not be construed in any way as limiting. Additionally, it is contemplated that although particular embodiments of the present disclosure may be disclosed or shown in the context of an IV set, such embodiments can be used in other fluid conveyance systems. Furthermore, various applications of such embodiments and modifications thereto, which may occur to those who are skilled in the art, are also encompassed by the general concepts described herein.

In accordance with some embodiments, the present disclosure includes various features and advantages of maintaining separation between a post and luer, thus minimizing the likelihood of damaging the post and/or the luer.

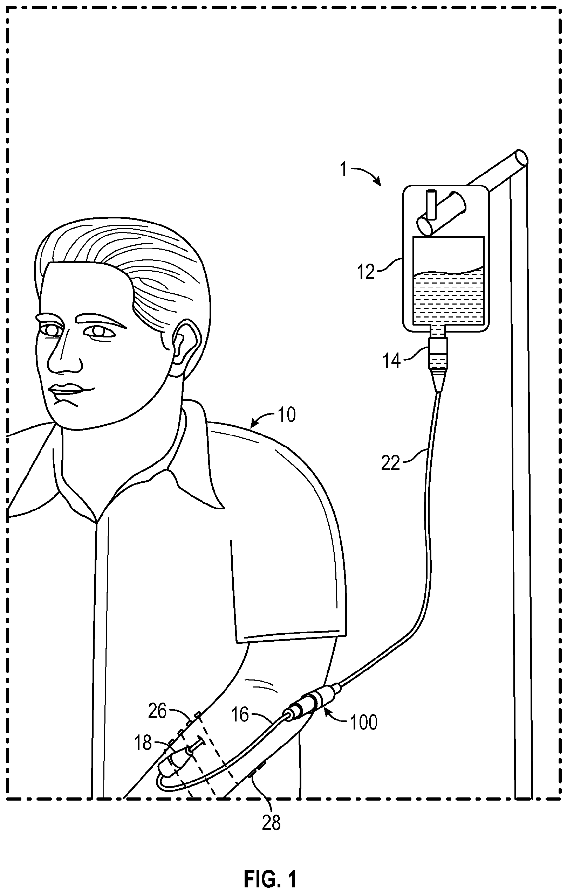

Referring now to the figures, FIG. 1 illustrates an IV set 1 coupled to a patient 10, in accordance with aspects of the present disclosure. The IV set 1 includes a medicament bag 12, a drip chamber 14, and tubing 22. The tubing 22 extends between the drip chamber 14 and a fluid connector assembly 100 of the IV set 1. To resist unintended dislodgement or disconnection of the tubing 16 or the catheter 18 from the patient, tape 26 is placed over the tubing 16 and the catheter 18, so that the tape 26 engages the tubing 16, the catheter 18, and the patient 10.

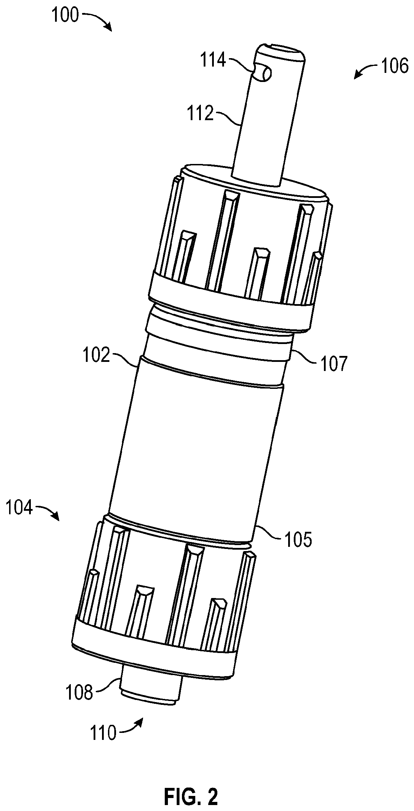

FIG. 2 illustrates a perspective view of a fluid connector assembly 100 for use with an IV set, in accordance with aspects of the present disclosure. The fluid connector assembly 100 is designed for use in medical applications, such as the IV set 1 (shown in FIG. 1) as well as other IV medical fluid delivery applications using catheters, including PIVC catheters, as non-limiting examples.

The fluid connector assembly 100 provides a regulated fluid path throughout the components of the fluid connector assembly 100. As shown, the fluid connector assembly 100 includes a housing 102 used as a central body to carry and/or connect with one or more components. The housing 102 may include a cylindrical, or generally cylindrical, body. Additionally, the housing 102 may include a hollow, or generally hollow, body that forms an internal volume to receive one or more components.

The fluid connector assembly 100 further includes connector 104 and a connector 106, each of which can couple, including mechanically couple, with the housing 102. As shown, the housing 102 includes an end 105 and an end 107, and the connector 104 and the connector 106 is connected to the end 105 and the end 107, respectively. The connectors 104 and 106 may be referred to as a first connector and a second connector, respectively. Also, the end 105 and the end 107 may be referred to as a first end and a second end, respectively. However, "first" and "second" may be interchangeable for the connectors 104 and the 106, as well as the ends 105 and 107. Also, each of the connectors 104 and 106 may be referred to as a medical connector. In some embodiments, the connector 106 is connected to a medical fluid, such as the medicament bag 12 (shown in FIG. 1). Further, in some embodiments, the connector 104 is connected to a catheter line (e.g., tubing 16 in FIG. 1) that delivers the medical fluid to a catheter, such as the catheter 18 (shown in FIG. 1). Also, each of the connectors 104 and 106 may take the form of a luer designed to prevent fluid leakage through their respective connections with the housing 102. In this regard, each of the connectors 104 and 106 may conform to standards established by the International Organization for Standards ("ISO") to improve patient safety, minimize medical fluid leakage, and reduce misconnection with other connection devices.

The connector 104 includes a post 108 that includes a channel fluidly connected to an opening (not shown in FIG. 2) at the end of the post 108. As a result, the fluid outlet 110 acts as a fluid transmission location for the fluid connector assembly 100. Also, the connector 106 includes a post 112 that includes an opening 114. The opening 114 acts as a fluid inlet of the fluid connector assembly 100. Accordingly, the opening 114 acts as a fluid receiving location for the fluid connector assembly 100. Each of the posts 108 and 112 can pass centrally, or at least approximately centrally, through the connectors 104 and 106, respectively. The posts 108 and 112 may be referred to as a first post and a second post, respectively. However, "first" and "second" may be used interchangeably. Also, each of the posts 108 and 112 includes a cylindrical, or generally cylindrical, shape. However, other shapes are possible.

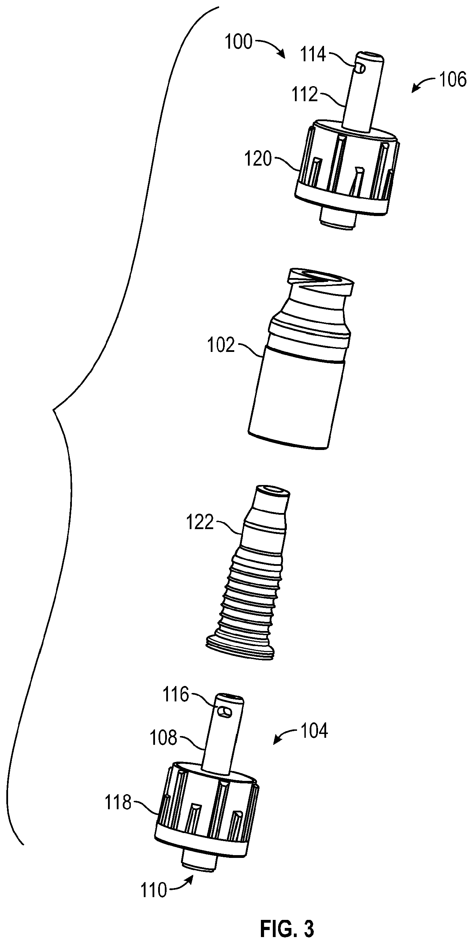

FIG. 3 illustrates an exploded view of the fluid connector assembly 100, showing additional features, in accordance with aspects of the present disclosure. The connector 104 includes an opening 116 formed in the post of the connector 104. The opening 116 represents a fluid inlet for the connector 104. The opening 116 is fluidly connected to the fluid outlet 110. Additionally, each of the connectors 104 and 106 include connector portions integrated with the respective posts. For example, the connector 104 includes a connector portion 118 and the connector 106 includes a connector portion 120. As shown in FIG. 3, the posts 108 and 112 extend through opposing ends of the connector portions 118 and 120, respectively.

Additionally, the fluid connector assembly 100 includes a compressible member 122. In some embodiments, the compressible member 122 includes a bellow that can elastically compress. Accordingly, the compressible member 122 can compress by an external force (or by multiple external forces) and subsequently return to its original, uncompressed state when the external force is removed. The compressible member 122 is designed to regulate fluid flow through the fluid connector assembly 100. Accordingly, the compressible member 122 acts as a valve for the fluid connector assembly 100. This will be shown in detail below.

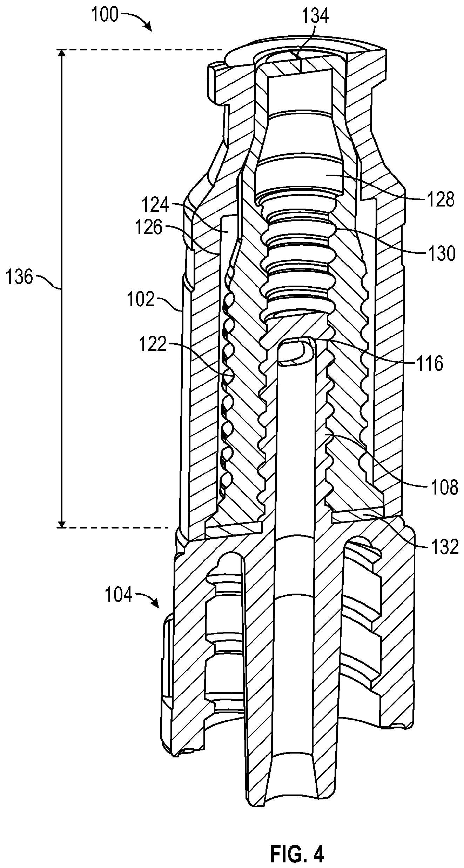

FIG. 4 illustrates a partial cross-sectional view of the fluid connector assembly 100, showing the connector 104 and the compressible member 122 positioned in the housing 102, in accordance with aspects of the present disclosure. Based on the hollow design, the housing 102 includes an internal volume 124 that forms a three-dimensional space or void in the housing 102, thus allowing the housing 102 to receive one or more components. For example, the compressible member 122 and a portion of the post 108 of the connector 104 are positioned in the internal volume 124. The housing 102 further includes an inner surface 126, or wall, that at least partially defines the internal volume 124 of the housing 102.

Additionally, a portion of the post 108 is located in the compressible member 122. In this regard, the compressible member 122 includes an internal volume 128 through which the portion of the post 108 lies. The compressible member 122 further includes an inner surface 130, or wall, that at least partially defines the internal volume 128 of the compressible member 122. Also, as shown in FIG. 4, the inner surface 130 of the compressible member 122 surrounds the post 108, including the opening 116 of the post 108. Moreover, the inner surface 130 of the compressible member 122 is in contact with the post 108. As a result of this relationship between the inner surface 130 and the opening 116, the compressible member 122 prevents fluid flow through the opening 116. In some embodiments, the compressible member 122 includes a septum 132 that provides a liquid-sealing structure that forms a seal between the post 108 and the compressible member 122 to prevent fluid leakage between the post 108 and the compressible member 122.

In some embodiments, the septum 132 can be integrated with the compressible member 122, and in some embodiments, the septum 132 may be separated from the compressible member 122. The compressible member 122 further includes a slit 134, or opening, that separates based on an applied force. As shown in FIG. 4, the slit 134 is closed and may resist fluid flow therethrough. However, when the connector 106 (shown in FIG. 3) is coupled with the housing 102, the post 112 of the connector 106 can engage the compressible member 122, causing the slit 134 to open based upon the applied force provided by the post 112.

Further, the compressible member 122 includes a dimension 136 that defines a lengthwise dimension of a major axis of the compressible member 122. When no external force is acting on the compressible member 122 to displace (e.g., compress) the compressible member 122, the lengthwise dimension of the compressible member 122 is defined by the dimension 136. Accordingly, the dimension 136 of the compressible member 122 represents an initial dimension, and the size and shape of the compressible member 122 shown in FIG. 4 represents an initial size and shape.

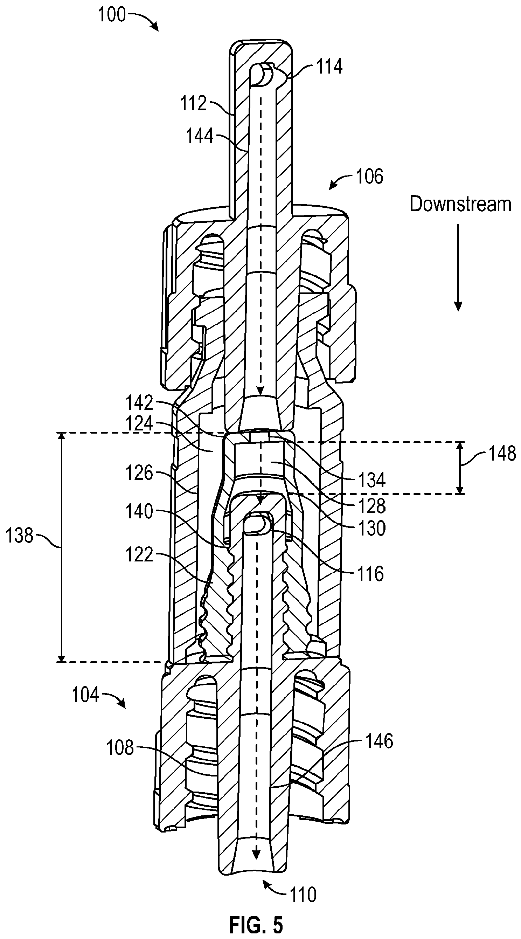

FIG. 5 illustrates a partial cross-sectional view of the fluid connector assembly 100, showing the connector 106 inserted into the housing 102, in accordance with aspects of the present disclosure. As shown, the post 112 of the connector 106 is at least partially disposed in the housing 102 and engages the compressible member 122, causing the compressible member 122 to displace. For example, the compressible member 122 compresses and reduces to a dimension 138 that is less than the dimension 136 (the original, uncompressed dimension shown in FIG. 4). Based on the displacement of the compressible member 122, each of the internal volume 128 and the inner surface 130 of the compressible member 122 transforms. For example, the internal volume 128 reduces and expands around the opening 116 of the post 108 of the connector 104, and the inner surface 130 of the compressible member 122 no longer engages the post 108 at a location surrounding the opening 116 of the post 108. Further, the displacement of the compressible member 122 causes the slit 134 of the compressible member 122 to open. As a result, fluid passing through the slit 134 of the compressible member 122 can further pass through the opening 116 of the post 108. Accordingly, in the displaced configuration of the compressible member 122, the opening 116 of the post 108 is fluidly connected to the slit 134 of the compressible member 122.

Based upon the engagement between the compressible member 122 and the connectors 104 and 106, several seals are formed. For example, a seal 140 is formed between the compressible member 122 and the post 108. The seal 140 prevents fluid entering the internal volume 128 of the compressible member 122 from passing through a location between the post 108 and the compressible member 122, and the fluid entering the compressible member 122 downstream passes through the opening 116. Also, a seal 142 is formed between the compressible member 122 and the post 112. The seal 142 prevents fluid entering a channel 144 of the post 112 of the connector 106 from passing through a location between the post 112 and the compressible member 122, and the fluid in the channel 144 passes through the slit 134. The seals 140 and 142 may be referred to as a first seal and a second seal, respectively. However, "first" and "second" may be interchangeable.

The arrows with dotted lines show a fluid path through the fluid connector assembly 100. When a medical fluid line is connected to the connector 106, the fluid enters the opening 114 (i.e., fluid inlet) of the connector 106. The fluid can then pass through the channel 144 of the post 112 and enter the compressible member 122 through the slit 134 of the compressible member 122. The fluid can then enter the opening 116 of the post 108, and subsequently pass through a channel 146 of the post 108. The fluid can exit the fluid connector assembly 100 through the fluid outlet 110 formed in the post 108.

Based on the fluid flow through the fluid connector assembly 100, the fluid connector assembly 100 provides neutral fluid displacement in which blood and/or other fluids is/are prevented, or at least substantially limited, from entering a catheter lumen (not shown) positioned in the connector 104 and fluidly connected to the fluid connector assembly 100 during a connector or disconnection between the fluid connector assembly 100 and the catheter lumen, or when the medical fluid line is connected or disconnected to the connector 106. However, unlike traditional neutral fluid displacement connector assemblies, the post 108 of the connector 104 is not inserted into the post 112 of the connector 106. Put another way, the post 112 of the connector 106 does not overlap the post 108 of the connector 104. As shown in FIG. 5, a gap 148, or separation, exists between the post 108 and the post 112. Accordingly, when the connectors 104 and 106 are coupled with the housing 102, the posts 108 and 112 are separated by the gap 148 in a direction along a longitudinal axis that passes through the housing 102, the connectors 104 and 106, and the compressible member 122.

Despite the gap 148, the seals 140 and 142 promote fluid flow through, and not around, the compressible member 122. For example, the seal 140 urges fluid exiting the compressible member 122 into the opening 116 of the post 108 and prevents fluid flowing outside the post 108 and into the internal volume 124 of the housing 102. Also, the seal 142 urges fluid exiting the channel 144 of the post 112 through the slit 134 of the compressible member 122 and prevents fluid flowing outside the compressible member 122 and into the internal volume 124 of the housing 102. As a result, fluid entering the fluid connector assembly 100 remains within the connector 106, the compressible member 122, and the connector 106, and does not contact the inner surface 126 of the housing 102. Based on the features and functionality, the fluid connector assembly 100, unlike positive fluid displacement connector assemblies, does not force fluid into the catheter lumen during a connection or disconnection event. Also, the fluid connector assembly 100, unlike negative fluid displacement connector assemblies, does not allow fluid back into the catheter lumen during a connection or disconnection event. Accordingly, the fluid connector assembly 100 includes a neutral displacement connector assembly, without overlapping posts.

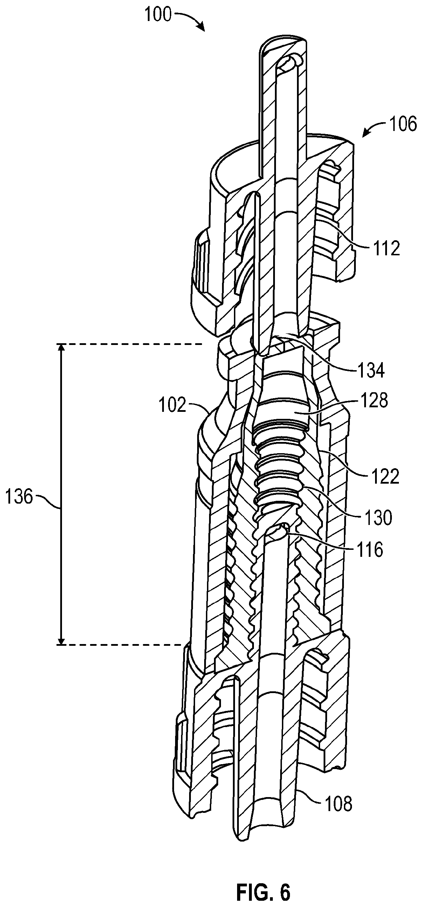

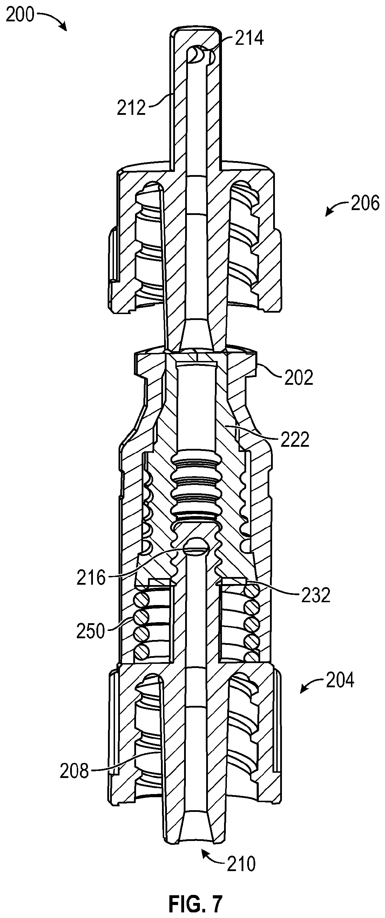

FIG. 6 illustrates a partial cross-sectional view of the fluid connector assembly 100, showing the connector 106 removed from the housing 102, in accordance with aspects of the present disclosure. Based on the removal of the connector 106, the compressible member 122 expands and returns to its original shape and size. In this regard, the compressible member 122 returns to having the dimension 136, representing the original lengthwise dimension of the compressible member 122 prior to displacement by the post 112 of the connector 106. Further, the slit 134 of the compressible member 122 closes after removal of the connector 106, and the slit 134 prevents fluid passage therethrough. Additionally, the internal volume 128 of the compressible member 122 returns to its prior, uncompressed volume and the inner surface 130 of the compressible member 122 covers the opening 116 of the post 108 to again seal the opening 116. FIG. 7 illustrates a partial cross-sectional view of an alternate embodiment of a fluid connector assembly 200, showing a biasing component 250 integrated with the fluid connector assembly 200, in accordance with aspects of the present disclosure. The fluid connector assembly 200 may include any structure(s), and associated feature(s), previously shown and described for fluid connector assemblies, along with additional features described herein. Accordingly, the fluid connector assembly 200 may provide neutral fluid displacement.

As shown, the fluid connector assembly 200 includes a housing 202, a connector 204, and a connector 206. The fluid connector assembly 200 further includes a compressible member 222 disposed in an internal volume (not labeled) of the housing 202. The connector 204 includes a post 208 that is at least partially disposed in the housing 202 and the compressible member 222. Also, the connector 206 includes a post 212 having an opening 214 that forms a fluid inlet for the fluid connector assembly 200. While the post 212 is not disposed in the housing 202 and does not displace the compressible member 222 in FIG. 7, the post 212 may engage and displace, or at least substantially displace, the compressible member 222 (for example, see post 112 and compressible member 122 in FIG. 5). Also, the connector 204 includes a post 208 having an opening 216. Also, the post 208 includes a fluid outlet 210 for the fluid connector assembly 200. Additionally, the compressible member 222 includes a septum 232 that provides a liquid-sealing structure that forms a seal between the post 208 and the compressible member 222 to prevent fluid leakage between the post 208 and the compressible member 222. In some embodiments, the septum 232 can be integrated with the compressible member 222, and in some embodiments, the septum 232 may be separated from the compressible member 222.

As shown, the biasing component 250 is located in the housing 202. The biasing component 250 may include a spring or other flexible structure designed to elastically compress. The biasing component 250 is designed to increase the stiffness of the septum 232 of the compressible member 222. By integrating the biasing component 250, the effective spring constant of the compressible member 222 increases, thus requiring an increased force acting on the compressible member 222 to displace the compressible member 222. In this regard, the biasing component 250 can increase the overall sealing capabilities of the compressible member 222, as the compressible member 222 increases in stiffness and provides a greater counter-resistance to displacement by, for example, the post 212 of the connector 206.

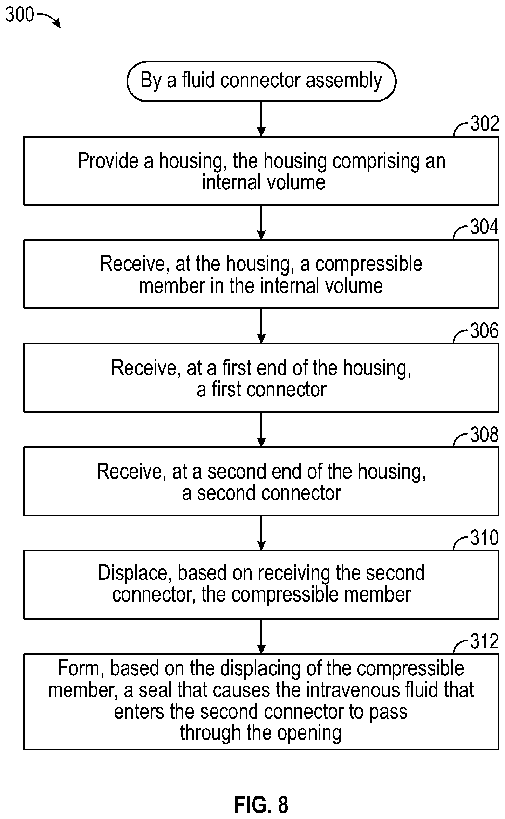

FIG. 8 illustrates a flowchart 300 showing a method for regulating an intravenous fluid, in accordance with aspects of the present disclosure. Fluid connector assemblies shown or described herein are capable of carrying out the steps of the method shown in the flowchart 300. In this regard, fluid connector assemblies with neutral fluid displacement may carry the steps of the method shown in the flowchart 300.

In step 302, a housing is provided. The housing may act as a central body for the fluid connector assembly. The housing may include an internal volume designed to receive, or at least partially receive, one or more components of the fluid connector assembly.

In step 304, a compressible member is received at the housing. Further, the compressible member is received in the internal volume of the housing. The compressible member may include a bellow. Further, the compressible member may elastically compress based on an external force and return to its original shape after the external force is removed.

In step 306, a first connector is received at a first end of the housing. The first connector may include a post and an opening formed in the post. The post, including the opening of the post, can be inserted into the housing, and also inserted into the compressible member. Also, the post of the first connector may form a fluid outlet for the fluid connector assembly.

In step 308, a second connector is received at a second end of the housing. The second connector may include a post that engages the compressible member. Also, the post of the second connector may include an opening that forms a fluid inlet for the fluid connector assembly.

In step 310, the compressible member is displaced based on receiving the second connector. The displacement of the compressible member may include a compression that causes a dimension (e.g., length) of the compressible member to reduce. However, based on the elastic features of the compressible member, the compressible member may return to its original size and shape when the second connector no longer engages the compressible member.

In step 312, a seal is formed based on the displacing of the compressible member. The seal causes the intravenous fluid entering the second connector to pass through the opening of the post of the first connector. Further, the seal promotes neutral fluid displacement by limiting or preventing blood and/or other fluids from entering a catheter lumen when a connection or disconnection with the fluid connector assembly occurs. Also, the fluid within the fluid connector assembly does not extend into the internal volume of the housing and does not contact the inner surface of the housing.

Also, based on the location of the posts of the first and second connectors within the housing, the posts do not overlap each other. Put another way, the post of the first connector ("first post") does not enter the post of the second connector ("second post"), and the second post does not surround the first post. In this regard, a gap, located along a longitudinal axis of the first and second posts, provides a separation between the first and second posts, thus minimizing the likelihood of contact between the posts that can cause damage to at least one of the first and second posts.

The features of the present disclosure provide a fluid connector assembly with a compressible member that can be displaced to form a fluid pathway therebetween. When a first and a second connector are coupled together with a housing that carries the compressible member, the compressible member can compress and allow fluid flow, while also providing multiple seals to prevent unwanted fluid leakage. However, if a connector that displaces the compressible member is removed, whether unintentionally or intentionally, the fluid pathway the fluid connector assembly closes or is obstructed to prevent fluid loss therefrom, as the compressible member returns to its uncompressed state and closes off the fluid pathway. The features of the present disclosure also provide that upon separation of the first and second connectors, any of the first and second connectors can be cleaned and disinfected, and the first and second connectors can be once again coupled together to cause the compressible member to form a fluid pathway therebetween.

### Illustration of Subject Technology as Clauses

The subject technology is illustrated, for example, according to various aspects described below. Various examples of aspects of the subject technology are described as numbered clauses (1, 2, 3, etc.) for convenience. These are provided as examples and do not limit the subject technology. It is noted that any of the dependent clauses may be combined in any combination, and placed into a respective independent clause, e.g., clause 1, clause 8 or clause 15. The other clauses can be presented in a similar manner.

### Further Considerations

In some embodiments, any of the clauses herein may depend from any one of the independent clauses or any one of the dependent clauses. In one aspect, any of the clauses (e.g., dependent or independent clauses) may be combined with any other one or more clauses (e.g., dependent or independent clauses). In one aspect, a claim may include some or all of the words (e.g., steps, operations, means or components) recited in a clause, a sentence, a phrase or a paragraph. In one aspect, a claim may include some or all of the words recited in one or more clauses, sentences, phrases or paragraphs. In one aspect, some of the words in each of the clauses, sentences, phrases or paragraphs may be removed. In one aspect, additional words or elements may be added to a clause, a sentence, a phrase or a paragraph. In one aspect, the subject technology may be implemented without utilizing some of the components, elements, functions or operations described herein. In one aspect, the subject technology may be implemented utilizing additional components, elements, functions or operations.

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

A reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention.

The word "exemplary" is used herein to mean "serving as an example or illustration." Any aspect or design described herein as "exemplary" is not necessarily to be construed as preferred or advantageous over other aspects or designs. **In** one aspect, various alternative configurations and operations described herein may be considered to be at least equivalent.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such an embodiment may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such a configuration may refer to one or more configurations and vice versa.

**In** one aspect, unless otherwise stated, all measurements, values, ratings, positions, magnitudes, sizes, and other specifications that are set forth in this specification, including in the claims that follow, are approximate, not exact. **In** one aspect, they are intended to have a reasonable range that is consistent with the functions to which they relate and with what is customary in the art to which they pertain.

**In** one aspect, the term "coupled" or the like may refer to being directly coupled. In another aspect, the term "coupled" or the like may refer to being indirectly coupled.

Terms such as "top," "bottom," "front," "rear" and the like if used in this disclosure should be understood as referring to an arbitrary frame of reference, rather than to the ordinary gravitational frame of reference. Thus, a top surface, a bottom surface, a front surface, and a rear surface may extend upwardly, downwardly, diagonally, or horizontally in a gravitational frame of reference.

Various items may be arranged differently (e.g., arranged in a different order, or partitioned in a different way) all without departing from the scope of the subject technology. Moreover, nothing disclosed herein is intended to be dedicated to the public regardless of whether such disclosure is explicitly recited in the claims. Furthermore, to the extent that the term "include," "have," or the like is used, such term is intended to be inclusive in a manner similar to the term "comprise" as "comprise" is interpreted when employed as a transitional word in a claim.

The Title, Background, Summary, Brief Description of the Drawings and Abstract of the disclosure are hereby incorporated into the disclosure and are provided as illustrative examples of the disclosure, not as restrictive descriptions. It is submitted with the understanding that they will not be used to limit the scope or meaning of the claims. In addition, in the Detailed Description, it can be seen that the description provides illustrative examples and the various features are grouped together in various embodiments for the purpose of streamlining the disclosure. This method of disclosure is not to be interpreted as reflecting an intention that the claimed subject matter requires more features than are expressly recited in each claim. Rather, as the following claims reflect, inventive subject matter lies in less than all features of a single disclosed configuration or operation. The following claims are hereby incorporated into the Detailed Description, with each claim standing on its own as a separately claimed subject matter.

## Claims

1. A fluid connector assembly (100), comprising:
a housing (102), comprising a first end, a second end, and an internal volume (124) fluidly connected to the first end and the second end, and a compressible member (122) disposed in the internal volume, the compressible member (122) comprising a cavity (128);
a first connector (104) secured with the housing (102) at the first end, the first connector comprising a first post (108) having a first post end located in the cavity, and an opening (116) formed in the first post, wherein the compressible member (122) extends around the opening (116); and
a second connector (106) comprising a second post (112), and when the second connector (106) is secured with the housing (102) at second end, the second post (112) displaces the compressible member (122) to form a fluid pathway between the opening (116) and the second connector (106), and a first seal (140) is formed between the first post (108), the compressible member (122), and the second post (112), such that a fluid entering the second connector (106) can pass through the opening (116), and a second post end is spaced apart from the first post end by a gap (148) in a direction along a longitudinal axis through the first connector (104) and the second connector (106).

2. The fluid connector assembly of Claim 1, wherein the second post (112) and the compressible member (122) form a second seal (142).

3. The fluid connector assembly of Claim 2, wherein the compressible member (122) comprises a slit (134) that opens and is sealed by the second seal (142).

4. The fluid connector assembly of Claim 2, wherein: the housing (102) comprises an inner surface (126), and the first seal (140) and the second seal (142) prevent the fluid from contacting the inner surface (126) of the housing.

5. The fluid connector assembly of Claim 1, wherein the first post (108) does not overlap the second post (112).

6. The fluid connector assembly of Claim 1, wherein the compressible member (122) is positioned between the first post (108) and the second post (112).

7. The fluid connector assembly of Claim 1, wherein the second connector (!06) compresses the compressible member (122) from a first dimension (136) to a second dimension (138) less than the first dimension.

8. The fluid connector assembly of Claim 3, wherein the slit (134) is located in the gap (148).

9. The fluid connector assembly of Claim 1, wherein:
the housing (102) comprises an inner wall the defines the internal volume,
and
a fluid entering the second connector is prevented from contacting the inner surface (126) of the housing based on the first seal (140) formed between the compressible member (122) and the first post (108), and a second seal (142) formed between the compressible member (122) and the second post (112).

10. A method of assembling a fluid connector assembly (100):
providing a housing (102), the housing comprising an internal volume (124);
receiving, at the housing (102), a compressible member (122) in the internal volume (124);
receiving, at a first end of the housing (102), a first connector (104), the first connector comprising a first post (108) and an opening (116) formed in the first post, wherein the compressible member (122) extends around the opening (116);
receiving, at a second end of the housing, a second connector (106) comprising a second post (112);
displacing, based on receiving the second connector (106), the compressible member (122) such that a gap (148) remains in an axial direction between a second post end of the second post (112) a first post end of the first post (108), and a fluid pathway is formed between the opening and the second connector; and
forming, based on the displacing of the compressible member (122), a seal between the first post (108), the compressible member (122), and the second post (112), such that the intravenous fluid that enters the second connector (106) can pass through the opening (116).

11. The method of Claim 10, wherein forming the seal comprises forming a first seal (140), and wherein the method further comprises forming, by the second connector and the compressible member (122), a second seal (142).

12. The method of Claim 11, further comprising directing, based on the first seal (140) and the second seal (142), the intravenous fluid though the compressible member (122) such that the intravenous fluid does not contact an inner surface (126) of the housing.

13. The method of Claim 11, wherein
receiving the first connector (104) and the second connector ()106 comprises maintaining a separation from the first post (108) and the second post (112).

14. The method of Claim 13, wherein maintaining the separation comprises positioning the second post (112) in a non-overlapping manner with the first post (108).

15. The method of Claim 13, wherein displacing the second connector comprises compressing the compressible member (122) from a first (136)dimension to a second dimension (138) less than the first dimension.

## Patentansprüche

1. Fluidkonnektoranordnung (100), aufweisend:
ein Gehäuse (102), aufweisend ein erstes Ende, ein zweites Ende und ein Innenvolumen (124), das mit dem ersten Ende und dem zweiten Ende fluidisch verbunden ist, und ein komprimierbares Element (122), das in dem Innenvolumen angeordnet ist, wobei das komprimierbare Element (122) einen Hohlraum (128) aufweist;
einen ersten Konnektor (104), der am ersten Ende an dem Gehäuse (102) befestigt ist, wobei der erste Konnektor einen ersten Stift (108), der ein erstes Stiftende aufweist, das sich in dem Hohlraum befindet, und eine in dem ersten Stift ausgebildete Öffnung (116) aufweist, wobei sich das komprimierbare Element (122) um die Öffnung (116) herum erstreckt; und
einen zweiten Konnektor (106), der einen zweiten Stift (112) aufweist, und wenn der zweite Konnektor (106) am zweiten Ende an dem Gehäuse (102) befestigt ist, verlagert der zweite Stift (112) das komprimierbare Element (122), um einen Fluidpfad zwischen der Öffnung (116) und dem zweiten Konnektor (106) zu bilden, und eine erste Abdichtung (140) wird zwischen dem ersten Stift (108), dem komprimierbaren Element (122) und dem zweiten Stift (112) gebildet, sodass ein in den zweiten Konnektor (106) eintretendes Fluid durch die Öffnung (116) hindurchtreten kann, und ein zweites Stiftende ist durch einen Spalt (148) in einer Richtung entlang einer Längsachse durch den ersten Konnektor (104) und den zweiten Konnektor (106) von dem ersten Stiftende beabstandet.

2. Fluidkonnektoranordnung nach Anspruch 1, wobei der zweite Stift (112) und das komprimierbare Element (122) eine zweite Abdichtung (142) bilden.

3. Fluidkonnektoranordnung nach Anspruch 2, wobei das komprimierbare Element (122) einen Schlitz (134) aufweist, der sich öffnet und durch die zweite Abdichtung (142) abgedichtet wird.

4. Fluidkonnektoranordnung nach Anspruch 2, wobei: das Gehäuse (102) eine Innenfläche (126) aufweist, und die erste Abdichtung (140) und die zweite Abdichtung (142) verhindern, dass das Fluid mit der Innenfläche (126) des Gehäuses in Kontakt kommt.

5. Fluidkonnektoranordnung nach Anspruch 1, wobei der erste Stift (108) den zweiten Stift (112) nicht überlappt.

6. Fluidkonnektoranordnung nach Anspruch 1, wobei das komprimierbare Element (122) zwischen dem ersten Stift (108) und dem zweiten Stift (112) positioniert ist.

7. Fluidkonnektoranordnung nach Anspruch 1, wobei der zweite Konnektor (106) das komprimierbare Element (122) von einer ersten Abmessung (136) auf eine zweite Abmessung (138) komprimiert, die kleiner als die erste Abmessung ist.

8. Fluidkonnektoranordnung nach Anspruch 3, wobei sich der Schlitz (134) in dem Spalt (148) befindet.

9. Fluidkonnektoranordnung nach Anspruch 1, wobei:
das Gehäuse (102) eine Innenwand aufweist, die das Innenvolumen definiert,
und
ein in den zweiten Konnektor eintretendes Fluid daran gehindert wird, mit der Innenfläche (126) des Gehäuses in Kontakt zu kommen, auf Grundlage der ersten Abdichtung (140), die zwischen dem komprimierbaren Element (122) und dem ersten Stift (108) gebildet ist, und einer zweiten Abdichtung (142), die zwischen dem komprimierbaren Element (122) und dem zweiten Stift (112) gebildet ist.

10. Verfahren zum Zusammenbauen einer Fluidkonnektoranordnung (100):
Bereitstellen eines Gehäuses (102), wobei das Gehäuse ein Innenvolumen (124) aufweist;
Aufnehmen eines komprimierbaren Elements (122) an dem Gehäuse (102) in dem Innenvolumen (124);
Aufnehmen eines ersten Konnektors (104) an einem ersten Ende des Gehäuses (102), wobei der erste Konnektor einen ersten Stift (108) und eine in dem ersten Stift ausgebildete Öffnung (116) aufweist, wobei sich das komprimierbare Element (122) um die Öffnung (116) herum erstreckt;
Aufnehmen eines zweiten Konnektors (106), der einen zweiten Stift (112) aufweist, an einem zweiten Ende des Gehäuses;
Verlagern des komprimierbaren Elements (122) auf Grundlage des Aufnehmens des zweiten Konnektors (106), sodass ein Spalt (148) in einer axialen Richtung zwischen einem zweiten Stiftende des zweiten Stifts (112) und einem ersten Stiftende des ersten Stifts (108) verbleibt und ein Fluidpfad zwischen der Öffnung und dem zweiten Konnektor gebildet wird; und
Bilden einer Abdichtung zwischen dem ersten Stift (108), dem komprimierbaren Element (122) und dem zweiten Stift (112) auf Grundlage des Verlagerns des komprimierbaren Elements (122), sodass das intravenöse Fluid, das in den zweiten Konnektor (106) eintritt, durch die Öffnung (116) hindurchtreten kann.

11. Verfahren nach Anspruch 10, wobei das Bilden der Abdichtung das Bilden einer ersten Abdichtung (140) umfasst, und wobei das Verfahren ferner das Bilden einer zweiten Abdichtung (142) durch den zweiten Konnektor und das komprimierbare Element (122) umfasst.

12. Verfahren nach Anspruch 11, ferner umfassend das Leiten des intravenösen Fluids durch das komprimierbare Element (122) auf Grundlage der ersten Abdichtung (140) und der zweiten Abdichtung (142), sodass das intravenöse Fluid nicht mit einer Innenfläche (126) des Gehäuses in Kontakt kommt.

13. Verfahren nach Anspruch 11, wobei
das Aufnehmen des ersten Konnektors (104) und des zweiten Konnektors (106) das Aufrechterhalten einer Trennung von dem ersten Stift (108) und dem zweiten Stift (112) umfasst.

14. Verfahren nach Anspruch 13, wobei das Aufrechterhalten der Trennung das Positionieren des zweiten Stifts (112) in nicht überlappender Weise mit dem ersten Stift (108) umfasst.

15. Verfahren nach Anspruch 13, wobei das Verlagern des zweiten Konnektors das Komprimieren des komprimierbaren Elements (122) von einer ersten Abmessung (136) auf eine zweite Abmessung (138), die kleiner als die erste Abmessung ist, umfasst.

## Revendications

1. Ensemble connecteur de fluide (100), comprenant :
un boîtier (102), comprenant une première extrémité, une seconde extrémité et un volume interne (124) relié fluidiquement à la première extrémité et à la seconde extrémité, et un élément compressible (122) disposé dans le volume interne, l'élément compressible (122) comprenant une cavité (128) ;
un premier connecteur (104) fixé au boîtier (102) au niveau de la première extrémité, le premier connecteur comprenant une première tige (108) ayant une première extrémité de tige située dans la cavité, et une ouverture (116) formée dans la première tige, dans lequel l'élément compressible (122) s'étend autour de l'ouverture (116) ; et
un second connecteur (106) comprenant une seconde tige (112), et lorsque le second connecteur (106) est fixé au boîtier (102) au niveau de la seconde extrémité, la seconde tige (112) déplace l'élément compressible (122) pour former un trajet de fluide entre l'ouverture (116) et le second connecteur (106), et une première étanchéité (140) est formée entre la première tige (108), l'élément compressible (122) et la seconde tige (112), de sorte qu'un fluide entrant dans le second connecteur (106) peut passer par l'ouverture (116), et une seconde extrémité de tige est espacée de la première extrémité de tige par un intervalle (148) dans une direction le long d'un axe longitudinal passant par le premier connecteur (104) et le second connecteur (106).

2. Ensemble connecteur de fluide selon la revendication 1, dans lequel la seconde tige (112) et l'élément compressible (122) forment une seconde étanchéité (142).

3. Ensemble connecteur de fluide selon la revendication 2, dans lequel l'élément compressible (122) comprend une fente (134) qui s'ouvre et est rendue étanche par la seconde étanchéité (142).

4. Ensemble connecteur de fluide selon la revendication 2, dans lequel : le boîtier (102) comprend une surface intérieure (126), et la première étanchéité (140) et la seconde étanchéité (142) empêchent le fluide d'entrer en contact avec la surface intérieure (126) du boîtier.

5. Ensemble connecteur de fluide selon la revendication 1, dans lequel la première tige (108) ne chevauche pas la seconde tige (112).

6. Ensemble connecteur de fluide selon la revendication 1, dans lequel l'élément compressible (122) est positionné entre la première tige (108) et la seconde tige (112).

7. Ensemble connecteur de fluide selon la revendication 1, dans lequel le second connecteur (106) comprime l'élément compressible (122) d'une première dimension (136) à une seconde dimension (138) inférieure à la première dimension.

8. Ensemble connecteur de fluide selon la revendication 3, dans lequel la fente (134) est située dans l'intervalle (148).

9. Ensemble connecteur de fluide selon la revendication 1, dans lequel :
le boîtier (102) comprend une paroi intérieure qui définit le volume interne, et
un fluide entrant dans le second connecteur est empêché d'entrer en contact avec la surface intérieure (126) du boîtier sur la base de la première étanchéité (140) formée entre l'élément compressible (122) et la première tige (108), et d'une seconde étanchéité (142) formée entre l'élément compressible (122) et la seconde tige (112).

10. Procédé d'assemblage d'un ensemble connecteur de fluide (100) :
fournir un boîtier (102), le boîtier comprenant un volume interne (124) ;
recevoir, au niveau du boîtier (102), un élément compressible (122) dans le volume interne (124) ;
recevoir, au niveau d'une première extrémité du boîtier (102), un premier connecteur (104), le premier connecteur comprenant une première tige (108) et une ouverture (116) formée dans la première tige, dans lequel l'élément compressible (122) s'étend autour de l'ouverture (116) ;
recevoir, au niveau d'une seconde extrémité du boîtier, un second connecteur (106) comprenant une seconde tige (112) ;
déplacer, sur la base de la réception du second connecteur (106), l'élément compressible (122) de telle sorte qu'un intervalle (148) subsiste dans une direction axiale entre une seconde extrémité de tige de la seconde tige (112) et une première extrémité de tige de la première tige (108), et un trajet de fluide est formé entre l'ouverture et le second connecteur ; et
former, sur la base du déplacement de l'élément compressible (122), une étanchéité entre la première tige (108), l'élément compressible (122) et la seconde tige (112), de sorte que le fluide intraveineux qui entre dans le second connecteur (106) peut passer par l'ouverture (116).

11. Procédé selon la revendication 10, dans lequel la formation de l'étanchéité comprend la formation d'une première étanchéité (140), et dans lequel le procédé comprend en outre la formation, par le second connecteur et l'élément compressible (122), d'une seconde étanchéité (142).

12. Procédé selon la revendication 11, comprenant en outre l'étape consistant à diriger, sur la base de la première étanchéité (140) et de la seconde étanchéité (142), le fluide intraveineux à travers l'élément compressible (122) de sorte que le fluide intraveineux n'entre pas en contact avec une surface intérieure (126) du boîtier.

13. Procédé selon la revendication 11, dans lequel
la réception du premier connecteur (104) et du second connecteur (106) comprend le maintien d'une séparation de la première tige (108) et de la seconde tige (112).

14. Procédé selon la revendication 13, dans lequel le maintien de la séparation comprend le positionnement de la seconde tige (112) de manière non chevauchante avec la première tige (108).

15. Procédé selon la revendication 13, dans lequel le déplacement du second connecteur comprend la compression de l'élément compressible (122) d'une première dimension (136) à une seconde dimension (138) inférieure à la première dimension.
